# EUROPEAN PATENT SPECIFICATION

(11) **EP 2 170 377 B1**
(45) Date of publication and mention of the grant of the patent: **04.03.2015**
(21) Application number: 07868328.1
(22) Date of filing: 13.08.2007
(51) Int. Cl.: A61K 39/108, A61K 39/145, A61K 39/39, A61K 39/00, C07K 14/425

(54) **MUTATED E. COLI HEAT-LABILE ENTEROTOXIN**
MUTIERTES HITZELABILES E. COLI ENTEROTOXIN
ENTEROTOXINE THERMOLABILE D'E. COLI AYANT MUTE

(30) Priority: 18.07.2007 US 779419
(43) Date of publication of application: 07.04.2010
(73) Proprietor: Development Center For Biotechnology, Xizhi City, 221 (TW)
(72) Inventor: HSU, Yu-Shen, Xizhi City 221 (TW); LIN, Young-Sun, Taipei City 114 (TW); YUAN, Ta-Tung, San Marino, California 91108 (US)
(74) Representative: Purdylucey Intellectual Property
(86) International application number: PCT/US2007/075801
(87) International publication number: WO 2009/011707

(56) References cited:
- WO-A1-01/70257
- US-A1- 2001 044 416
- US-A1- 2008 102 078
- CIEPLAK W ET AL: "Site-Directed Mutagenic Alteration of Potential Active-site Residues of the A Subunit of Escherichia coli Heat-labile Enterotoxin, Evidence for a catalytic role for glutamic acid 112", JOURNAL OF BIOLOGICAL CHEMISTRY, AMERICAN SOCIETY FOR BIOCHEMISTRY AND MOLECULAR BIOLOGY, INC, US, vol. 270, no. 51, 22 December 1995 (1995-12-22), pages 30545-30550, XP008122555, ISSN: 0021-9258
- DOMENIGHINI M. ET AL.: 'Microcorrespondence: Identification of Errors Among Database Sequence Entries and Comparison of Correct Amino Acid Sequences for the Heat-Labile Enterotoxins of Escherichia coli and Vibrio Cholerae' MOLECULAR MICROBIOLOGY vol. 15, no. 6, 1995, pages 1165 - 1167, XP000654469
- CIEPLAK W. ET AL.: 'Site-Directed Mutagenic Alteration of Potential Active-site Residues of the Subunit of Escherichia coli Heat-labile Enterotoxin' THE JOURNAL OF BIOLOGICAL CHEMISTRY vol. 270, no. 51, 1995, pages 30545 - 30550, XP008122555
- HARFORD S. ET AL.: 'Inactivation of the Escherichia coli Heat-Labile Enterotoxin by in vitro Mutagenesis of the A Subunit Gene' EUR. J. BIOCHEM. vol. 183, no. 2, 1989, pages 311 - 316, XP002070940
- STREATFIELD S.J. ET AL.: 'Intermolecular Interactions between the A and B Subunits of Heat-Labile Enterotoxin from Escherichia coli Promote Holotoxin Assembly and Stability in vivo' PROC. NATL. SCI. vol. 89, 1992, pages 12140 - 12144, XP008122557
- 'Non-Polio Enterovirus Infections' CDC FAT SHEET, [Online] 05 September 2006, XP008137343 Retrieved from the Internet: <URL:http://www.cdc.gov/ncidod/dvrd/revb/en terovirus/non-polio:_entero.htm>
- SVENNERHOLM A. ET AL.: 'Progress in Vaccine Development Against Helicobacter pylori' FEMS IMMUNOL. MED. MICROBIOL. vol. 50, 2007, pages 146 - 156, XP008122559
- 'Hepatitis C' CDC FACT SHEET, [Online] 06 March 2008, XP008134289 Retrieved from the Internet: <URL:http://www.cdc.gov/hepatitis>
- GIRARD M.P. ET AL.: 'A Review of Vaccine Research and Development: The Human Immunodeficiency Virus (HIV)' VACCINE vol. 24, 2006, pages 4062 - 4081, XP005421550

## Description

### CROSS-REFERENCE TO RELATED APPLICATIONS

This application claims priority from United States Patent Application No. 11/779,419 filed July 18.

### BACKGROUND

Enterotoxigenic *Escherichia coli* strains cause diarrhea in humans and domestic animals by producing two types of enterotoxins, i.e., heat-labile toxin (LT) and heat-stable toxin (ST) (Hofstra et al., 1984, J. Bio. Chem. 259:15182-15187). LT is functionally, structurally, and immunologically related to cholera toxin (CT) (Clements et al., 1978, Infect. Immun. 21:1036-1039). LT and CT are synthesized as holotoxin molecules composed of five identical subunits B and an enzymatically active subunit A (AB₅) (Spangler, 1992, Microbio. Rev. 56(4):622-647). The B pentamer binds ganglioside GM1 in the membrane of intestinal epithelial cells or any other cell that contains GM1 (van Heyningen, 1974, Science 183:656-657). Following binding of subunits B to GM1 on the cell surface, subunit A is inserted into cytosal and proteolytically cleaved and reduced at its single disulfide bond to produce an enzymatically active A1 peptide and a smaller A2 peptide (Fishman PH, 1982, J. Membr. Biol. 69:85-97; Mekalanos et al., 1979, J. Biol. Chem. 254:5855-5861; Moss et al., 1981, J. Biol. Chem. 256:12861-12865). The A1 peptide is capable of binding NAD and catalyzing the ADP-ribosylation of Gsα, a GTP-binding regulatory protein associated with adenylate cyclase (Spangler, 1992, Microbio. Rev. 56(4):622-647). The resulting increase in cAMP eventually leads to the release of electrolytes and fluids from affected cells (Cheng et al., 2000, Vaccine 18:38-49).

LT has been shown to function as mucosal adjuvant and induce immune responses against mucosally co-administered antigens (Clements et al., 1988, Vaccine 6:269-277; Elson CO. 1989, Immunol. Today 146:29-33; Spangler, 1992, Microbio. Rev. 56(4):622-647). However, the high toxicity of wild-type LT has limited its clinical use. Thus, it is desirable to generate mutated LTs having reduced toxicity while retaining immunogenicity.

The term "E. coli heat-labile enterotoxin" or "LT" used herein refers to a heat-labile enterotoxin produced by any enterotoxigenic E. coli strain. The term "E. coli heat-labile enterotoxin subunit A" or "LT_{A}" refers to subunit A of LT. It includes both precursor LT_{A}, which contains a signal peptide, and mature LT_{A} which has the signal peptide removed.

### SUMMARY

This invention is based on the unexpected discovery that an LT containing a mutated LT_{A} exhibits reduced toxicity compared to its wild type counterpart while retaining immunogenicity. This mutated LT_{A} has an amino acid substitution at the position corresponding to position 61 of a wild-type LT_{A}, whose amino acid sequence, SEQ ID NO:5, is shown below.

Accordingly, this invention features an isolated polypeptide including a mutated LT_{A} that contains an amino acid residue other than S, T, and F, at the position corresponding to position 61 of SEQ ID NO:5. The substituting amino acid residue can be D, E, H, I, K, L, N, P, Q, R, Y or W. It can be a naturally occurring amino acid or a non-naturally occurring amino acid, e.g., a D-amino acid or a β-amino acid. In one example, the LT_{A} has the amino acid sequence of SEQ ID NO:2, 4, 8 or 10. An LT containing this mutated LT_{A} exhibits reduced toxicity, i.e., < 10⁻⁵-fold that of a wild-type LT containing SEQ ID NO:5.

In another aspect, this invention features a vaccine containing an antigen and the mutated LT_{A} described above. The vaccine can further include LT subunit B, which forms with the mutated LT_{A} whole LT protein. The antigen can be derived from either a bacterium or a virus.

In yet another aspect, this invention features an isolated nucleic acid including a nucleotide sequence that encodes the above-described LT_{A} mutant. In one example, the nucleotide sequence is SEQ ID NOs: 1, 3, 7, or 9. Also within the scope of this invention is a vector including the just-described nucleic acid and a transformed cell containing the vector.

The details of one or more embodiments of the invention are set forth in the description below. Other features, objects, and advantages of the invention will be apparent from the description and from the claims.

### DETAILED DESCRIPTION

A detoxified LT, e.g., containing a mutated LT_{A}, is a desired vaccine adjuvant due to its high immunogenecity. The mutated LT_{A} of this invention, designed to achieve this purpose, is made by introducing an amino acid substitution in an LT_{A} at a position corresponding to the position 61 of SEQ ID NO:5. LT_{A} produced by different enterotoxigenic E. coli strains are highly homologous. Thus, by comparing the amino acid sequence of an LT_{A} with SEQ ID NO:5, a skilled artisan can find out which position in this LT_{A} corresponds to position 61 of SEQ ID NO:5. Just like the LT_{A} of SEQ ID NO:5, almost all wild-type LT_{A}s include a serine at the position corresponding to position 61 of SEQ ID NO:5. An amino acid that is different from serine in, e.g., size or polarity, can be used as a substitutient. Examples of the substitutient amino acids include hydrophobic amino acid residues (e.g., I and L), charged amino acid residues (e.g., D, E, K, R, and H), or amino acid residues with bulky side chains (e.g., N, Q, Y, and W). Proline also can be used as a substitutient as it generally alters the local structure of a polypeptide. These substitutient amino acids include non-naturally occurring amino acids, e.g., D-amino acids or β-amino acids.

The LT_{A} mutant of this invention can be prepared by methods well known in the art. For example, the mutant is produced by a recombinant method as follows. A cDNA that encodes a wild-type LT_{A} is isolated from an enterotoxigenic E. coli strain and subjected to site-directed mutagenesis to produce a cDNA encoding the desired LT_{A} mutant. See Ho et al., Gene, 77:51-59,1989. The cDNA bearing the mutation is then inserted into an expression vector for transforming cells. Finally, the LT_{A} mutant produced in the transformed cells is purified and assembled with LT subunit B to form whole LT protein.

The toxicity of an LT containing an LT_{A} mutant described above can be assessed using assays such as the Y-1 adrenal cell assay. See Cheng et al., Vaccine, 18:38-49, 2000.

The LT_{A} mutant of this invention can be used as an adjuvant in a vaccine. The vaccine (i.e., a human vaccine or a veterinary vaccine) can contain an antigen and the LT_{A} mutant itself or an LT containing the LT_{A} mutant. The antigen can be derived from a bacterium, e.g., Streptococcus pyogenes, Streptococcus pneumoniae, Neiseria gonorrhoea, Neisseria meningitides, Corynebacterium diphtheriae, Clostridium botulinum, Clostridium perfringens, Clostridium tetani, Haemophilus influenzae, Klebsiella pneumoniae, Klebsiella ozaenae, Staphylococcus aureus, Vibrio cholerae, Escherichia coli, Pseudomonas aeruginosa, Campylobacter jejuni, Aeromonas hydrophila, Bacillus cereus, Yersinia enterocolitica, Yersinia pestis, Salmonella typhimurium, Treponema pallidum, Borrelia vincentii, Borrelia burgdorferi, Mycobacterium tuberculosis, Pneumocystis carinii, Mycoplasma spp., Rickettsia prowazeki, Chlamydia spp., Helicobacter pylori. It also can be derived from a virus, e.g., influenza, herpes simplex virus, human immunodeficiency virus, cytomegalovirus, hepatitis c virus, delta hepatitis virus, poliovirus, hepatitis A virus, hepatitis B virus, Epstein-barr virus, varicella zoster virus, respiratory syncytial virus, enterovirus or Japanese encephalitis virus.

The vaccine can further contain a pharmaceutically acceptable carrier such as phosphate buffered saline or a bicarbonate solution. The carrier is selected on the basis of the mode and route of administration, and standard pharmaceutical practice. Suitable pharmaceutical carriers and diluents, as well as pharmaceutical necessities for their use, are described in Remington's Pharmaceutical Sciences.

Methods for preparing vaccines are generally well known in the art, as exemplified by U.S. Patents. 4,601,903; 4,599,231; 4,599,230; and 4,596,792. Vaccines may be prepared as injectables, as liquid solutions or emulsions. The antigen and the LT_{A} mutant or the LT containing it may be mixed with physiologically acceptable excipients, which may include, water, saline, dextrose, glycerol, ethanol, and combinations thereof. The vaccine may further contain minor amounts of auxiliary substances such as wetting or emulsifying agents, or pH buffering agents to enhance the effectiveness of the vaccines. Vaccines may be administered parenterally, by injection subcutaneously or intramuscularly. Alternatively, other modes of administration including suppositories, oral, or topical formulations may be desirable. For suppositories, binders and carriers may include, for example, polyalkalene glycols or triglycerides. Oral formulations may include normally employed incipients such as, for example, pharmaceutical grades of saccharine, cellulose, magnesium carbonate and the like. These compositions take the form of solutions, suspensions, tablets, pills, capsules, sustained release formulations or powders.

The vaccine is administered in a manner compatible with the dosage formulation, and in an amount that is therapeutically effective, protective and immunogenic. The quantity to be administered depends on the subject to be treated, including, for example, the capacity of the individual's immune system to synthesize antibodies, and if needed, to produce a cell-mediated immune response. Precise amounts of active ingredient required to be administered depend on the judgment of the practitioner. However, suitable dosage ranges are readily determinable by one skilled in the art and may be of the order of micrograms of the polypeptide of this invention. Suitable regimes for initial administration and booster doses are also variable, but may include an initial administration followed by subsequent administrations. The dosage of the vaccine may also depend on the route of administration and varies according to the size of the host.

The specific examples below are to be construed as merely illustrative, and not limitative of the remainder of the disclosure in any way whatsoever. Without further elaboration, it is believed that one skilled in the art can, based on the description herein, utilize the present invention to its fullest extent.

### Example 1: Constructing genes encoding wild type LT_{A} and LT mutated LT_{A}

A 1.8-kb DNA fragment of an LT gene, including both subunit A and subunit B, was isolated from human entero-toxigenic E. coli H10407 and cloned into pBluescript II KS(-) vector (pBluescript-LThWT). The nucleotide sequence (SEQ ID NO:6) of the LT gene (encoding both Subunits A and B) and the amino acid sequence (SEQ ID NO:5) of subunit A of this LT are shown below:

Nucleotide sequence (SEQ ID NO:6) of LT (Subunit A: 1-777; Subunit B: 774-1148)

atgaaaaata taactttcat tttttttatt ttattagcat cgccattata tgcaaatggc 60
gacaaattat accgtgctga ctctagaccc ccagatgaaa taaaacgttc cggaggtctt
120
atgcccagag ggcataatga gtacttcgat agaggaactc aaatgaatat taatctttat
180
gatcacgcga gaggaacaca aaccggcttt gtcagatatg atgacggata tgtttccact
240

tctcttagtt tgagaagtgc tcacttagca ggacagtcta tattatcagg atattccact
300
tactatatat atgttatagc gacagcacca aatatgttta atgttaatga tgtattaggc
360
gtatacagcc ctcacccata tgaacaggag gtttctgcgt taggtggaat accatattct
420
cagatatatg gatggtatcg tgttaatttt ggtgtgattg atgaacgatt acatcgtaac
480
agggaatata gagaccggta ttacagaaat ctgaatatag ctccggcaga ggatggttac
540
agattagcag gtttcccacc ggatcaccaa gcttggagag aagaaccctg gattcatcat
600
gcaccacaag gttgtggaaa ttcatcaaga acaattacag gtgatacttg taatgaggag
660
acccagaatc tgagcacaat atatctcagg aaatatcaat caaaagttaa gaggcagata
720
ttttcagact atcagtcaga ggttgacata tataacagaa ttcggaatga attatgaata
780
aagtaaaatg ttatgtttta tttacggcgt tactatcctc tctatgtgca tacggagctc
840
cccagtctat tacagaacta tgttcggaat atcgcaacac acaaatatat acgataaatg
900
acaagatact atcatatacg gaatcgatgg caggcaaaag agaaatggtt atcattacat
960
ttaagagcgg cgcaacattt caggtcgaag tcccgggcag tcaacatata gactcccaaa
1020
aaaaagccat tgaaaggatg aaggacacat taagaatcac atatctgacc gagaccaaaa
1080
ttgataaatt atgtgtatgg aataataaaa cccccaattc aattgcggca atcagtatg
1140
aaaactag

Amino acid sequence (SEQ ID NO:5) of mature LTA
NGDKLYRADS RPPDEIKRSG GLMPRGHNEY FDRGTQMNIN LYDHARGTQT GFVRYDDGYV 60
STSLSLRSAH LAGQSILSGY STYYIYVIAT APNMFNVNDV LGVYSPHPYE QEVSALGGIP
120
YSQIYGWYRV NFGVIDERLH RNREYRDRYY RNLNIAPAED GYRLAGFPPD HQAWREEPWI
180
HHAPQGCGNS SRTITGDTCN EETQNLSTIY LRKYQSKVKR QIFSDYQSEV DIYNRIRNEL
240

Various LT_{A} mutants were constructed using a site-directed mutagenesis method (Ho et al., 1989, Gene 77, 51-59). More specifically, LT_{A} mutants, including LT_{A}(S61K), LT_{A}(S61R), LT_{A}(S61H), LT_{A}(S61Y) and LT_{A}(S61F) were constructed by replacing the serine at position 61 of SEQ ID NO:5 with K, R, H, Y, and F, respectively. The following oligonucleotide primers were used to construct these mutants: LT_{A}(S61K) [5' TCT CAA ACT AAG AGA AGT TTT AAC ATA TCC GTC ATC ATA 3'](SEQ ID NO:11), LT_{A}(S61R) [5' ACT TCT CAA ACT AAG AGA AGT TCT AAC ATA TCC GTC ATC 3'](SEQ ID NO:12), LT_{A}(S61F) [5' ACT TCT CAA ACT AAG AGA AGT GAA AAC ATA TCC GTC ATC 3'](SEQ ID NO:13), LT_{A}(S61Y) [5' ACT TCT CAA ACT AAG AGA AGT ATA AAC ATA TCC GTC ATC 3'](SEQ ID NO:14), LT_{A}(S61H) [5' ACT TCT CAA ACT AAG AGA AGT ATG AAC ATA TCC GTC ATC 3'](SEQ ID NO:15). The nucleotide sequences and amino acid sequences of these LT_{A} are shown below:

Nucleotide sequence (SEQ ID NO:1) of LT_{A}(S61K)
atgaaaaata taactttcat tttttttatt ttattagcat cgccattata tgcaaatggc 60
gacaaattat accgtgctga ctctagaccc ccagatgaaa taaaacgttc cggaggtctt
120
atgcccagag ggcataatga gtacttcgat agaggaactc aaatgaatat taatctttat
180
gatcacgcga gaggaacaca aaccggcttt gtcagatatg atgacggata tgttaaaact
240
tctcttagtt tgagaagtgc tcacttagca ggacagtcta tattatcagg atattccact
300
tactatatat atgttatagc gacagcacca aatatgttta atgttaatga tgtattaggc
360
gtatacagcc ctcacccata tgaacaggag gtttctgcgt taggtggaat accatattct
420
cagatatatg gatggtatcg tgttaatttt ggtgtgattg atgaacgatt acatcgtaac
480
agggaatata gagaccggta ttacagaaat ctgaatatag ctccggcaga ggatggttac
540
agattagcag gtttcccacc ggatcaccaa gcttggagag aagaaccctg gattcatcat
600
gcaccacaag gttgtggaaa ttcatcaaga acaattacag gtgatacttg taatgaggag
660
acccagaatc tgagcacaat atatctcagg aaatatcaat caaaagttaa gaggcagata
720
ttttcagact atcagtcaga ggttgacata tataacagaa ttcggaatga attatga
777

Amino acid sequence (SEQ ID NO:2) of LTA(S61K)
NGDKLYRADS RPPDEIKRSG GLMPRGHNEY FDRGTQMNIN LYDHARGTQT GFVRYDDGYV 60
KTSLSLRSAH LAGQSILSGY STYYIYVIAT APNMFNVNDV LGVYSPHPYE QEVSALGGIP
120
YSQIYGWYRV NFGVIDERLH RNREYRDRYY RNLNIAPAED GYRLAGFPPD HQAWREEPWI
180
HHAPQGCGNS SRTITGDTCN EETQNLSTIY LRKYQSKVKR QIFSDYQSEV DIYNRIRNEL
240

Nucleotide sequence (SEQ ID NO:3) of LT_{A}(S61R)

atgaaaaata taactttcat tttttttatt ttattagcat cgccattata tgcaaatggc 60
gacaaattat accgtgctga ctctagaccc ccagatgaaa taaaacgttc cggaggtctt
120

atgcccagag ggcataatga gtacttcgat agaggaactc aaatgaatat taatctttat
180
gatcacgcga gaggaacaca aaccggcttt gtcagatatg atgacggata tgttagaact
240
tctcttagtt tgagaagtgc tcacttagca ggacagtcta tattatcagg atattccact
300
tactatatat atgttatagc gacagcacca aatatgttta atgttaatga tgtattaggc
360
gtatacagcc ctcacccata tgaacaggag gtttctgcgt taggtggaat accatattct
420
cagatatatg gatggtatcg tgttaatttt ggtgtgattg atgaacgatt acatcgtaac
480
agggaatata gagaccggta ttacagaaat ctgaatatag ctccggcaga ggatggttac
540
agattagcag gtttcccacc ggatcaccaa gcttggagag aagaaccctg gattcatcat
600
gcaccacaag gttgtggaaa ttcatcaaga acaattacag gtgatacttg taatgaggag
660
acccagaatc tgagcacaat atatctcagg aaatatcaat caaaagttaa gaggcagata
720
ttttcagact atcagtcaga ggttgacata tataacagaa ttcggaatga attatga
777

Amino acid sequence (SEQ ID NO:4) of LTA (S61R)
NGDKLYRADS RPPDEIKRSG GLMPRGHNEY FDRGTQMNIN LYDHARGTQT GFVRYDDGYV 60
RTSLSLRSAH LAGQSILSGY STYYIYVIAT APNMFNVNDV LGVYSPHPYE QEVSALGGIP
120
YSQIYGWYRV NFGVIDERLH RNREYRDRYY RNLNIAPAED GYRLAGFPPD HQAWREEPWI
180
HHAPQGCGNS SRTITGDTCN EETQNLSTIY LRKYQSKVKR QIFSDYQSEV DIYNRIRNEL
240

Nucleotide sequence (SEQ ID NO:7) of LT_{A}(S61H)
atgaaaaata taactttcat tttttttatt ttattagcat cgccattata tgcaaatggc 60
gacaaattat accgtgctga ctctagaccc ccagatgaaa taaaacgttc cggaggtctt
120
atgcccagag ggcataatga gtacttcgat agaggaactc aaatgaatat taatctttat
180
gatcacgcga gaggaacaca aaccggcttt gtcagatatg atgacggata tgttcatact
240
tctcttagtt tgagaagtgc tcacttagca ggacagtcta tattatcagg atattccact
300
tactatatat atgttatagc gacagcacca aatatgttta atgttaatga tgtattaggc
360
gtatacagcc ctcacccata tgaacaggag gtttctgcgt taggtggaat accatattct
420
cagatatatg gatggtatcg tgttaatttt ggtgtgattg atgaacgatt acatcgtaac
480
agggaatata gagaccggta ttacagaaat ctgaatatag ctccggcaga ggatggttac
540
agattagcag gtttcccacc ggatcaccaa gcttggagag aagaaccctg gattcatcat
600

gcaccacaag gttgtggaaa ttcatcaaga acaattacag gtgatacttg taatgaggag
660
acccagaatc tgagcacaat atatctcagg aaatatcaat caaaagttaa gaggcagata
720
ttttcagact atcagtcaga ggttgacata tataacagaa ttcggaatga attatga
777

Amino acid sequence (SEQ ID NO:8) of LTA(S61H)

NGDKLYRADS RPPDEIKRSG GLMPRGHNEY FDRGTQMNIN LYDHARGTQT GFVRYDDGYV 60
HTSLSLRSAH LAGQSILSGY STYYIYVIAT APNMFNVNDV LGVYSPHPYE QEVSALGGIP
120
YSQIYGWYRV NFGVIDERLH RNREYRDRYY RNLNIAPAED GYRLAGFPPD HQAWREEPWI
180
HHAPQGCGNS SRTITGDTCN EETQNLSTIY LRKYQSKVKR QIFSDYQSEV DIYNRIRNEL
240

Nucleotide sequence (SEQ ID NO:9) of LT_{A}(S61Y)
atgaaaaata taactttcat tttttttatt ttattagcat cgccattata tgcaaatggc 60
gacaaattat accgtgctga ctctagaccc ccagatgaaa taaaacgttc cggaggtctt
120
atgcccagag ggcataatga gtacttcgat agaggaactc aaatgaatat taatctttat
180
gatcacgcga gaggaacaca aaccggcttt gtcagatatg atgacggata tgtttatact
240
tctcttagtt tgagaagtgc tcacttagca ggacagtcta tattatcagg atattccact
300
tactatatat atgttatagc gacagcacca aatatgttta atgttaatga tgtattaggc
360
gtatacagcc ctcacccata tgaacaggag gtttctgcgt taggtggaat accatattct
420
cagatatatg gatggtatcg tgttaatttt ggtgtgattg atgaacgatt acatcgtaac
480
agggaatata gagaccggta ttacagaaat ctgaatatag ctccggcaga ggatggttac
540
agattagcag gtttcccacc ggatcaccaa gcttggagag aagaaccctg gattcatcat
600
gcaccacaag gttgtggaaa ttcatcaaga acaattacag gtgatacttg taatgaggag
660
acccagaatc tgagcacaat atatctcagg aaatatcaat caaaagttaa gaggcagata
720
ttttcagact atcagtcaga ggttgacata tataacagaa ttcggaatga attatga
777

Amino acid sequence (SEQ ID NO:10) of LTA(S61Y)

NGDKLYRADS RPPDEIKRSG GLMPRGHNEY FDRGTQMNIN LYDHARGTQT GFVRYDDGYV 60
YTSLSLRSAH LAGQSILSGY STYYIYVIAT APNMFNVNDV LGVYSPHPYE QEVSALGGIP
120
YSQIYGWYRV NFGVIDERLH RNREYRDRYY RNLNIAPAED GYRLAGFPPD HQAWREEPWI
180

HHAPQGCGNS SRTITGDTCN EETQNLSTIY LRKYQSKVKR QIFSDYQSEV DIYNRIRNEL
240

For comparison, another LT mutant, LTp(S63K), derived from EWD299 (Dallas et al., 1979, J. Bacteriol. 139, 850-859) was also constructed by replacement of serine at amino acid position 63 of subunit A with lysine.

### Example 2: Preparing wild type LT and LT containing mutated LT_{A}

pBluescript II KS(-) vectors containing native or mutant LT genes, including genes for both subunit A and subunit B, were transformed into *E. coli* HB101. The native and mutant LT's were purified from cultures grown overnight in a 3-liter flask containing L-broth supplemented with 100µg of ampicillin per ml. The cells were harvested by centrifugation, resuspended in TEAN buffer (0.2 M NaCl, 50 mM Tris, 1 mM EDTA and 3 mM NaN3, pH 7.4), and lysed with microfluidizer (Microfluidics Corporation, USA). After the lysates were clarified by centrifugation, the LT was fractionated by adding solid ammonium sulfate to 65% saturation. The preparation was then suspended in TEAN buffer, dialyzed thoroughly against the same buffer and used as the crude LT. The crude LT was subjected to chromatography on Immobilized D-galactose (Pierce, Rockford, IL) columns equilibrated with TEAN buffer at 4°C (Uesaka et al., 1994, Microbial Pathogenesis 16:71-76). Native and mutant LT's were eluted with 0.3 M galactose in TEAN. Each purified toxin was dialyzed against PBS buffer for biological and immunological assays.

The purified wild-type and mutant LT were separated by SDS-PAGE, wherein the molecular weight of LT subunit A was about 28 to 29 kDa, and the molecular weight of LT subunit B was about 12 to 13 kDa. The yields of the entire LT containing LT_{A}(S61K), i.e., LTh(S61K), and LT_{A}(S61R), i.e., LTh(S61R) were similar to the LT containing the LT_{A} of SEQ ID NO:5.

AB₅ heterohexamers and B₅ pentamers of the LT were separated by pH 3-10 isoelectric focusing gel (invitrogen). Intensity of AB5 and B5 protein bands was assayed by UVIBand software (UVItec Limited) to calculate the percentage of the AB₅ heterohexamers and the B₅ pentamers (The isoelectric point (pI) value of AB₅ was between 8.0 and 7.8, and the pI value of B₅ was between 8.3 and 8.1), the results are listed in Table 1.

**Table 1. Preparation of wild-type LT and LT containing mutated LT_{A}**

| *E. coli* heat-labile enterotoxin (LT) | The ratio of AB5/AB5+B5 (%) |
|---|---|
| LTh(WT) | 91(%) |
| LTh(S61K) | 92(%) |
| LTh(S61R) | 85(%) |
| LTh(S61H) | 60(%) |
| LTh(S61F) | 70(%) |
| LTh(S61Y) | 57(%) |

| | |
|---|---|
| The percentages of AB₅ of purified LTh(S61K) and LTh(S61R) were about 90-100%. | |

### Example 3: Determining effect of wild-type LT and LT containing mutated LT_{A} on intracellular cAMP levels

Caco-2 cells (ATCC HTB-37) were maintained in MEM-α medium supplemented with 20% FBS in 24-well plate at a concentration of 5×104 cells per well, grown to near confluency, and incubated in MEM-α containing 1% FBS and 1mM 3-isobutyl-1-methylxanthine (IBMX) for 30 min in 5% CO2 prior to addition of toxins (Grant et al., 1994, Infection and Iimmunity 62: 4270-4278). Native or mutant LT was added to each well and incubated for 4 hours. The cells were then washed twice with cold PBS. Intracellular cAMP was extracted by adding 200 µl of 0.1 N HCl to each well and incubated at room temperature for 15 minutes. Supernatant of cell lysates were collected following addition of 0.1 N NaOH to each well. (Cheng et al., 2000, Vaccine 18: 38-49; Park et al., 1999, Experimental and Molecular Medicine 31: 101-107). cAMP was measured with a cAMP enzyme immunoassay kit (Assay designs; Correlate-EIA). Results obtained from this example show that LTh(S61K) did not increase the concentration of intracellular cAMP.

### Example 4: Determining toxicity of LT containing mutated LT_{A} using Y1 adrenal tumor cell assay

In this study, LT samples, including wild-type LT, LT active site mutant (h61K), and LT subunit B complex, B5, were evaluated for enterotoxic effect. Mouse Y-1 adrenal tumor cells (ATCC CCL-79) maintained in Ham's F12 media supplemented with 15% horse serum, 2.5% fetal bovine serum, 2mM L-glutamine, and 1.5 g/L sodium bicarbonate were seeded in 96-well flat-bottom plates at a concentration of 2 x 104 cells per well (200ul/well) at 37°C in 5% CO2 for 48 hrs. Cells in 96-well flat-bottom plates were washed twice with 1x PBS (pH 7.4) and then treated with serially diluted LT samples (10 µg/200 µl ∼ 10-10 µg/200 µl) at 37°C in 5% CO2 overnight. Cells were examined by light microscopy for typical cell rounding 24 hrs after toxin treatment. Activity is defined as the minimum concentration of the toxin required to initiate cell rounding (ECi) or the toxin concentration required for 50% cell rounding (EC50). See David et al., 1975, Infection and Immunity, 11:334-336; Cheng et al., 1999, Vaccine 18:38-49. The toxicity of LTh(S61K), LTh(S61R), LTh(S61H) is significantly lower compared to wild-type LT, i.e., 10⁻⁶ versus 1. See Table 2 below. The toxicity of LTh(S61F) is also lower (i.e.,10⁻⁵), but the reduction is not as significant as the other mutants.

**Table 2 Toxicity of LT containing wild-type or mutated LT_{A}**

| *E. coli* heat-labile enterotoxin (LT) | The amount of LT (pg/well) | Toxic intensity (wild type LT is 1) |
|---|---|---|
| LTh(WT) | 0.1 | 1 |
| LTh(S61K) | 100,000 | 10⁻⁶ |
| LTh(S61R) | 100,000 | 10⁻⁶ |
| LTh(S61H) | 100,000 | 10⁻⁶ |
| LTh(S61F) | 10,000 | 10⁻⁵ |
| LTh(S61Y) | 100,000 | 10⁻⁶ |
| LTh(S63K) | 10,000 | 10⁻⁵ |

### Example 5: Determining toxicity of LT containing mutated LT_{A} by rabbit ileal loop assay

The assay was performed as previously described (Giannelli et al., 1997, Infection and Immunity 65: 331-334; Giuliani et al., 1998, J. Exp. Med. 187:1123-1132). The New Zealand adult rabbits, ∼2.5 Kg each, were used for this assay. Loops, each 5 cm long, were made by starting at the end of the rabbit's small intestines and moving toward the stomach. 0.5 ml samples with various amounts of LT or LT mutants were injected intro each loop and then the abdomen was closed. After 18 hours, the liquid accumulated in each loop was collected and measured. The experiment was performed three times and the results expressed in milliliters per centimeter. Results from this experiment show that 500µg of LTh(S61K) only accumulated 1ml fluid in the rabbit ileal loop, and the volume of the accumulated fluid was similar to native control. When 0.1µg of wild-type LT was used, the volume of accumulated fluid of wild-type LT was considerably larger than that of LTh(S61K). In addition, the fluid accumulation of other LTp(S63K) also considerably larger than that of LTh(S61K).

### Example 6: Determining adjuvant effect of LT containing mutated LT_{A} in intranasal immunization

Deactivated Influenza virus, A/Puerto Rico/8/34 (H1N1) (PR8) (ATCC VR-95), was used in this example. The viral particles were prepared as previously described in (Aitken et al., 1980, Eur.J. Biochem. 107:51-56; Gallagher et al., 1984, J. Clin. Microbiol. 20:89-93; Johansson et al., 1989, J. Virol. 63:1239-1246). Briefly, the virus was propagated in the allantoic cavity of 10-day-old embryonated hen's eggs at 35°C for two days. The allantoic fluid from eggs infected with PR8 was first centrifuged at low-speed and then centrifuged at 96,000xg for 1 hour to precipitate viral particles, which were resuspended in phosphate-buffered saline (PBS). These particles were loaded onto a 30-60% sucrose density gradient and centrifuged for 5 hours at 96,000xg. The fraction containing the virus was collected and diluted with PBS. The virus was further pelleted at 96,000xg for 1 hour and resuspended in PBS. Purified virus was treated with 0.025% formalin at 4°C for a week. Protein concentration was measured and standardized based on the optical density, Bio-Rad Protein Assay, and the haemagglutinin (HA) content was determined by SDS-polyacryamide gel electrophoresis (Oxford et al., 1981, J. Biol. Stand. 9:483-491).

Female BALB/c mice, between 6 and 8 weeks old, were obtained from Taiwan National Laboratory Animal Center. Groups consisting of five of mice each were immunized intranasally with 25 µl PBS containing 20 µg of inactivated influenza virus (flu.Ag) alone or in combination with 8 µg of native or mutant LT under anesthesia. The mice were re-immunized 3 weeks later. Control mice were given PBS under the same condition. Two weeks after the final immunization, mice in each group were sacrificed to obtain serum and conduct haemagglutination inhibition (HI) assay. Significantly enhanced HI titers were detected in mice intranasally immunized with the inactivated virus vaccine in combination with LTh(S61K) or LTp(S63K) when compared with mice intranasally immunized with inactivated virus vaccine alone. The HI titer of LTh(S61K) in combination with the inactivated virus was substantially increased. The HI titer of LTh(S61K) in combination with the virus is 813, similar to that of LTh(S63K) in combination with the virus but larger than that of the virus alone.

### Example 7: Determining adjuvant effect of LT containing mutated LT_{A} in intramuscular immunization

The procedure carried out in Example 6 was repeated except that the immunization utilizes intramuscular delivery. Intramuscular vaccines were prepared in 50 µl PBS containing 10 µg of inactivated virus vaccine alone or in combination with 4 µg of native or mutant LT and were injected into the posterior thigh muscle. The immunization and sampling programs were performed as described. The HI titer of LTh(S61K) in combination with the inactivated virus was substantially increased. The HI titer of LTh(S61K) in combination with the virus was 512, which is significantly higher than that of wild type LT in combination with the virus or that of the virus alone.

### OTHER EMBODIMENTS

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

From the above description, one skilled in the art can easily ascertain the essential characteristics of the present invention, and without departing from the spirit and scope thereof, can make various changes and modifications of the invention to adapt it to various usages and conditions.

## Claims

1. An isolated polypeptide comprising an E. coli heat-labile enterotoxin subunit A, the *E. coli* heat-labile enterotoxin subunit A having an amino acid sequence selected from a group consisting of SEQ ID NOs: 2, 4, 8, and 10, wherein an E. coli heat-labile enterotoxin containing the subunit A having an amino acid sequence selected from the group consisting of SEQ ID NOs: 2, 4, 8 and 10 has a reduced toxicity when compared to a wild-type E. coli heat-labile enterotoxin containing SEQ ID NO:5.

2. A vaccine comprising an antigen and an adjuvant, the adjuvant being an E. coli heat-labile enterotoxin subunit A, the mutated *E. coli* heat-labile enterotoxin subunit A having an amino acid sequence selected from a ground consisting of SEQ ID NOs: 2, 4, 8, and 10, wherein an E. coli heat-labile enterotoxin containing the subunit A having an amino acid sequence selected from a group consisting of SEQ ID NOs: 2, 4, 8 has a reduced toxicity when compared to a wild-type E. coli heat-labile enterotoxin containing SEQ ID NO:5.

3. The vaccine of Claim 2, wherein the antigen is from a bacterium or a virus.

4. The vaccine of Claim 3, wherein the bacterium is selected from a group consisting of Streptococcus pneumoniae, Escherichia coli, Helicobacter pylori, Neisseria meningitidis, and Haemophilus influenza b, and wherein the virus is selected from a group consisting of Influenza virus, human immunodeficiency virus, hepatitis A virus, hepatitis B virus, hepatitis C virus, human papillomavirus, enterovirus, and rotavirus.

5. The vaccine of any of Claims 2 to 4, further comprising LT subunit B, wherein the subunit A mutant and the subunit B form whole E. coli heat-labile enterotoxin.

6. An isolated nucleic acid comprising a nucleotide sequence encoding an E. coli heat-labile enterotoxin subunit A, the *E. coli* heat-labile enterotoxin subunit A having an amino acid sequence selected from a group consisting of SEQ ID NOs:2, 4, 8, and 10, wherein an E. coli heat-labile enterotoxin containing the subunit A having an amino acid sequence selected from a group consisting of SEQ ID NOs: 2, 4, 8 has reduced toxicity when compared to a wild-type E. coli heat-labile enterotoxin containing SEQ ID NO:5.

7. The nucleic acid of Claim 6, wherein the nucleotide sequence is selected from a group consisting of SEQ ID NOs:1, 3, 7, and 9.

8. A vector comprising the nucleic acid of any of Claims 6 or 7.

9. A transformed cell comprising the vector of claim 8.

## Patentansprüche

1. Isoliertes Polypeptid, das eine hitzelabile Enterotoxin-Untereinheit A von E. coli umfasst, wobei die hitzelabile Enterotoxin-Untereinheit A von *E. coli* eine Aminosäuresequenz aufweist, die aus der Gruppe ausgewählt ist, die aus SEQ.-ID Nr. 2, 4, 8 und 10 besteht, worin ein hitzelabiles Enterotoxin von E. coli, das die Untereinheit A enthält, die eine Aminosäuresequenz aufweist, die aus der Gruppe ausgewählt ist, die aus SEQ.-ID Nr. 2, 4, 8 und 10 besteht, eine verringerte Toxizität im Vergleich zu einem hitzelabilen Enterotoxin von E. coli des Wildtyps, das die SEQ.-ID Nr. 5 enthält, aufweist.

2. Impfstoff, der ein Antigen und ein Adjuvans umfasst, wobei das Adjuvans eine hitzelabile Enterotoxin-Untereinheit A von E. coli ist, wobei die mutierte hitzelabile Enterotoxin-Untereinheit A von *E. coli* eine Aminosäuresequenz aufweist, die aus der Gruppe ausgewählt ist, die aus SEQ.-ID Nr. 2, 4, 8 und 10 besteht, worin ein hitzelabiles Enterotoxin von E. coli, das die Untereinheit A enthält, die eine Aminosäuresequenz aufweist, die aus der Gruppe ausgewählt ist, die aus SEQ.-ID Nr. 2, 4, 8 besteht, eine verringerte Toxizität im Vergleich zu einem hitzelabilen Enterotoxin von E. coli des Wildtyps, das die SEQ.-ID Nr. 5 enthält, aufweist.

3. Impfstoff nach Anspruch 2, worin das Antigen aus einem Bakterium oder einem Virus stammt.

4. Impfstoff nach Anspruch 3, worin das Bakterium aus einer Gruppe ausgewählt ist, die aus Folgenden besteht: Streptococcus pneumoniae, Escherichia coli, Helicobacter pylori, Neisseria meningitidis und Haemophilus influenza b, und worin das Virus aus einer Gruppe ausgewählt ist, die aus Folgenden besteht: Influenza-Virus, humanem Immundefizienz-Virus, Hepatitis-A-Virus, Hepatitis-B-Virus, Hepatitis-C-Virus, humanem Papillomavirus, Enterovirus und Rotavirus.

5. Impfstoff nach einem der Ansprüche 2 bis 4, der weiter LT-Untereinheit B umfasst, worin der Untereinheit-A-Mutant und die Untereinheit B das gesamte hitzelabile Enterotoxin von E. coli bilden.

6. Isolierte Nukleinsäure, die eine Nukleotidsequenz umfasst, die eine hitzelabile Enterotoxin-Untereinheit A von E. coli kodiert, wobei die hitzelabile Enterotoxin-Untereinheit A von *E. coli* eine Aminosäuresequenz aufweist, die aus einer Gruppe ausgewählt ist, die aus SEQ.-ID Nr. 2, 4, 8 und 10 besteht, worin ein hitzelabiles Enterotoxin von E. coli, das die Untereinheit A enthält, die eine Aminosäuresequenz aufweist, die aus einer Gruppe ausgewählt ist, die aus SEQ.-ID Nr. 2, 4, 8 besteht, eine verringerte Toxizität im Vergleich zu einem hitzelabilen Enterotoxin von E. coli des Wildtyps, das die SEQ.-ID Nr. 5 enthält, aufweist.

7. Nukleinsäure nach Anspruch 6, worin die Nukleotidsequenz aus einer Gruppe ausgewählt ist, die aus SEQ.-ID Nr. 1, 3, 7 und 9 besteht.

8. Vektor, der die Nukleinsäure nach einem der Ansprüche 6 oder 7 umfasst.

9. Transformierte Zelle, die den Vektor nach Anspruch 8 umfasst.

## Revendications

1. Polypeptide isolé comprenant la sous-unité A d'une entérotoxine thermolabile d'*E*. *coli,* la sous-unité A de l'entérotoxine thermolabile d'*E*. *coli* ayant une séquence d'acides aminés sélectionnée dans le groupe consistant en les SÉQ ID N° : 2, 4, 8 et 10, dans lequel une entérotoxine thermolabile d'*E*. *coli* contenant la sous-unité A dont la séquence d'acides aminés est sélectionnée dans le groupe consistant en les SÉQ ID N° : 2, 4, 8 et 10 a une toxicité plus basse que l'entérotoxine thermolabile d'E. *coli* de type sauvage qui contient la SÉQ ID N° : 5.

2. Vaccin comprenant un antigène et un adjuvant, l'adjuvant étant la sous-unité A d'une entérotoxine thermolabile d'E. *coli* et la sous-unité A d'une forme mutée de l'entérotoxine thermolabile d'*E*. *coli* ayant une séquence d'acides aminés sélectionnée dans le groupe consistant en les SÉQ ID N° : 2, 4, 8 et 10, dans lequel une entérotoxine thermolabile d'E. *coli* contenant la sous-unité A dont la séquence d'acides aminés est sélectionnée dans le groupe consistant en les SÉQ ID N° : 2, 4, 8 a une toxicité plus basse que l'entérotoxine thermolabile *d'E. coli* de type sauvage qui contient la SÉQ ID N° : 5.

3. Vaccin de la revendication 2, dans lequel l'antigène dérive d'une bactérie ou d'un virus.

4. Vaccin de la revendication 3, dans lequel la bactérie est sélectionnée dans un groupe consistant en *Streptococcus pneumoniae, Escherichia coli, Helicobacter pylori, Neisseria meningitidis* et *Haemophilus influenzae* type B et dans lequel le virus est sélectionné dans un groupe consistant en un virus de la grippe, le virus de l'immunodéficience humaine, le virus de l'hépatite A, le virus de l'hépatite B, le virus de l'hépatite C, le papillomavirus humain, un entérovirus et un rotavirus.

5. Vaccin de l'une quelconque des revendications 2 à 4 qui comprend également une sous-unité B de LT, dans lequel la forme mutée de la sous-unité A et la sous-unité B constituent ensemble une entérotoxine thermolabile d'E. *coli* entière.

6. Acide nucléique isolé qui comprend une séquence nucléotidique codant pour la sous-unité A d'une entérotoxine thermolabile d'E. *coli,* la sous-unité A de l'entérotoxine thermolabile d'E. *coli* ayant une séquence d'acides aminés sélectionnée dans le groupe consistant en les SÉQ ID N° : 2, 4, 8 et 10, dans lequel l'entérotoxine thermolabile d'E. *coli* contenant une sous-unité A dont la séquence d'acides aminés est sélectionnée dans le groupe consistant en les SÉQ ID N° : 2, 4, 8 a une toxicité plus basse que l'entérotoxine thermolabile d'*E*. *coli* de type sauvage qui contient la SÉQ ID N° : 5.

7. Acide nucléique de la revendication 6, dans lequel la séquence nucléotidique est sélectionnée dans le groupe consistant en les SÉQ ID N° : 1, 3, 7 et 9.

8. Vecteur comprenant l'acide nucléique de l'une quelconque des revendications 6 ou 7.

9. Cellule transformée comprenant le vecteur de la revendication 8.
